# EUROPÄISCHE PATENTSCHRIFT

(11) **EP 0 333 661 B1**
(45) Veröffentlichungstag und Bekanntmachung des Hinweises auf die Patenterteilung: **27.04.1994**
(21) Anmeldenummer: 89810188.6
(22) Anmeldetag: 09.03.1989
(51) Int. Cl.: C07D 405/04, C07F 1/02

(54) **Verfahren zur Herstellung eines Pyrrol-Derivats**
Process for the preparation of a pyrole derivative
Procédé pour la préparation d'un dérivé de pyrrole

(30) Priorität: 18.03.1988 CH 1052/88
(43) Veröffentlichungstag der Anmeldung: 20.09.1989
(73) Patentinhaber: CIBA-GEIGY AG, 4002 Basel (CH)
(72) Erfinder: Schaub, Bruno, Dr., CH-2822 Courroux (CH); Känel, Hans-Ruedi, Dr., CH-4416 Bubendorf (CH); Ackermann, Peter, Dr., CH-4148 Pfeffingen (CH)

(56) Entgegenhaltungen:
- EP-A- 0 082 681
- EP-A- 0 206 999
- DE-A- 3 601 285
- JOURNAL OF ORGANOMETALLIC CHEMISTRY Band 136, Nr. 2, März 1977, Seiten 139-146, Lausanne, CH; S. CABIDDU et al.: "Metalation Reactions III. The Action of n-Butyllithium on 1,3-Benzodioxole and 1,3-Benzoxathiole Derivatives".

## Beschreibung

Die vorliegende Erfindung betrifft ein Verfahren zur Herstellung von 3-(2,2-Difluorbenzodioxol-4-yl)-4-cyanopyrrol der Formel I 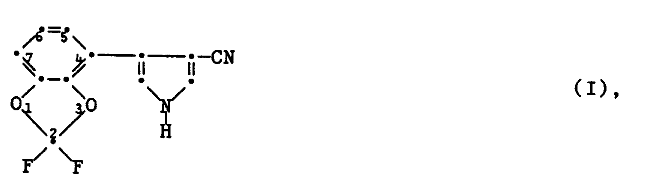
ferner die als Zwischenprodukte dieses Verfahrens auftretenden 4-Metallo-2,2-difluorbenzodioxole, sowie ein Verfahren zur Herstellung dieser Zwischenprodukte.

Aus der DE-PS 2,927,480 ist bekannt, dass N-Acyl-3-phenyl-4-cyanopyrrole eine fungizide Wirksamkeit aufweisen. In der EP-A 206999 ist das 3-(2,2-Difluor-benzodioxol-4-yl)-4-cyanopyrrol als Fungizid beschrieben.

Ein Verfahren zur Herstellung von 3-Phenyl-4-cyanopyrrolen ausgehend von α-Cyano-zimtsäuren (oder aus geeigneten Estern dieser Säure) durch Umsetzung mit einem substituierten Methyl-isocyanid wurde in der DE-OS 3,601,285 beschrieben.

Der Nachteil dieses Verfahrens ist, dass die den α-Cyanozimtsäuren als Ausgangsprodukte zugrundeliegenden Benzaldehyde bei gewissen Kernsubstitutionen nur schwer zugänglich sind. In solchen Fällen erweist sich ein auf dieser Reaktionsfolge aufbauendes Verfahren als sehr schwer durchführbar und unwirtschaftlich.

Ziel der vorliegenden Erfindung ist ein vom technischen und wirtschaftlichen Gesichtspunkt aus verbessertes und allgemein brauchbares Verfahren zur Herstellung von 3-(2,2-Difluorbenzodioxol-4-yl)-4-cyanopyrrol.

Es wurde nun gefunden, dass man 3-(3,2-Difluorbenzodioxol-4-yl)-4-cyanopyrrol der Formel I 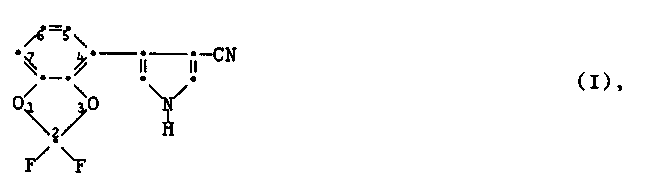
überraschenderweise sehr leicht im Eintopfverfahren herstellen kann, wenn man ein 4-Metallo-2,2-difluorbenzodioxol der Formel III, wobei Me ein Metall ausgewählt aus der Gruppe bestehend aus Li, Na, K, Cs, Mg, Ca, Zn, Cd, Cu, Pd, Ni, Al, Si, Sn, Ti und Zr, n die Wertigkeit dieses Metalls und Y einen anionischen Rest, ausgewählt aus der Gruppe bestehend aus Halogenid, Nitrat, Sulfat, Phosphat, Acetat, Formiat, C₁-C₁₀-Alkyl^{⊖}, C₆-C₁₄-Aryl^{⊖}, C₁-C₁₀-Alkoxy^{⊖}, NH₂^{⊖}, C₁-C₁₀-HydrocarbylNH^{⊖}, Di(C₁-C₁₀-hydrocarbyl)N^{⊖} und H^{⊖} bedeuten, in einem polaren Lösungsmittel oder in einem polaren Lösungsmittelgemisch, zunächst mit einem ungesättigten Nitril der Formel IV zur Reaktion bringt, 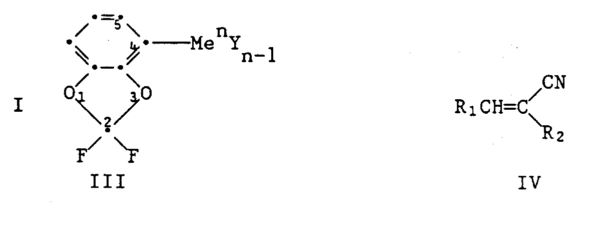
worin
- R₁: Halogen, C₁-C₄-Alkoxy, C₆-C₁₀-Aryloxy, Di-C₁-C₄-alkylamino, C₁-C₄-Alkylsulfonyloxy, C₆-C₁₀-Arylsulfonyloxy oder C₁-C₄-Alkylcarbonyloxy und
- R₂: einen C₁-C₄-Alkoxycarbonylrest bedeuten, und dann mit einem Isocyanid der Formel V reagieren lässt,

R₃-SO₂CH₂NC, (v),

worin R₃ ein offenkettiger oder cyclischer, unsubstituierter C₁-C₁₀-Kohlenwasserstoff ist.

Es wurde weiterhin gefunden und ist gleichfalls Gegenstand vorliegender Erfindung, dass man die Verbindungen der Formel IIIa sehr einfach und regioselektiv herstellen kann, wenn man 2,2-Difluorbenzodioxol der Formel II in einem polaren Lösungsmittel oder in einem polaren Lösungsmittelgemisch mit einer Metallverbindung Me'ⁿY'ₙ bei -70°C bis +150°C zur Reaktion bringt, 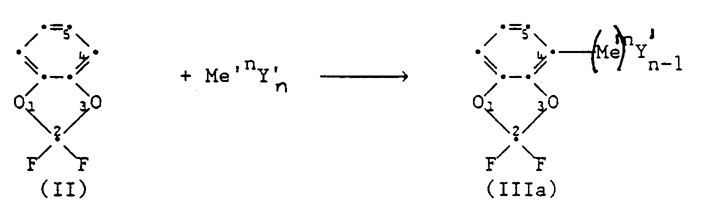
wobei Me' ein Metall ausgewählt aus der Gruppe bestehend aus Li, Na, K, Cs, Mg und Ca, n die Wertigkeit dieses Metalls und Y' einen anionischen Rest ausgewählt aus der Gruppe bestehend aus C₁-C₁₀-Alkyl^{⊖}, C₆-C₁₄-aryl^{⊖}, NH₂^{⊖}, C₁-C₁₀-HydrocarbylNH^{⊖}, Di(C₁-C₁₀-hydrocarbyl)N^{⊖} und H^{⊖}, bedeuten.

Die Reaktion wird vorzugsweise in einem Temperaturbereich von -25° bis +80°C durchgeführt.

Es kommen als Lösungsmittel z.B. Kohlenwasserstoffe (wie Petrolether, Toluol, Hexan, Heptan u.a.) und Ether (wie Diethylether, Dioxan, Tetrahydrofuran) neben den weiter unten aufgeführten in Frage. Werden bei der Metallierung unpolare oder schwach polare Lösungsmittel verwendet, so ist zur Erzielung und Gewährleistung einer hohen Regioselektivität der Einsatz von Komplexierungsmitteln notwendig oder zumindest vorteilhaft.

Unter den Verfahrensbedingungen bevorzugt ist die Verwendung einer komplexbildenden Verbindung, die ausgewählt ist aus einer Gruppe bestehend aus tert.Aminen, cyclischen Harnstoffen, Ethern und N-substituierten Säureamiden. Unter diesen sind Hexamethylphosphorsäuretriamid, Ethylenglykoldimethyläther, 1,3-Dimethyl-2-imidazolon, Dimethylethylenharnstoff, Dimethylpropylenharnstoff und N,N,N',N'-Tetramethylethylendiamin besonders bevorzugt.

Die durch dieses Verfahren herstellbaren Verbindungen der Formel IIIa sind auch Gegenstand der vorliegenden Erfindung.

Uebliche in der organischen Chemie beschriebene Metallierungsverfahren von Aromaten verlaufen in der Regel unselektiv und führen zu einem Gemisch isomerer metallorganischer Verbindungen. Es ist daher überraschend, dass die Metallierung von 2,2-Difluorbenzodioxol selektiv in der 4-Position erfolgt.

Die Gewinnung von Verbindungen der Formel III gelingt durch Ummetallierung einer Verbindung der Formel IIIa 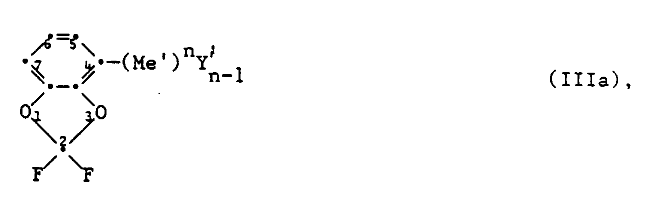
mit einer Verbindung der Formel MeⁿYₙ, wobei Me' ein von Me verschiedenes Metall bedeutet. Die Bedeutung des Metalls Me' und seiner Verbindungen beschränkt sich im wesentlichen auf Metalle der Alkali- und Erdalkaligruppe. Bevorzugt sind Li, Na, K, Cs und Mg, besonders bevorzugt ist Lithium. Diese Ummetallierung ist ebenfalls Gegenstand der vorliegenden Erfindung. Im allgemeinen lassen sich Alkalimetall- und Erdalkalimetall-Derivate des 2,2-Difluorbenzodioxols durch direkte Metallierung herstellen, während Metalle der übrigen Gruppen zweckmässig aus diesen durch Ummetallierung gewonnen werden. Die durch Ummetallierung oder Direktmetallierung erhältlichen 4-Metallo-2,2-difluorbenzodioxol-Derivate der Formel III sind auch Gegenstand vorliegender Erfindung, insbesondere jene, bei denen das Metall für Alkali- und Erdalkali-Metalle oder für Zink, Cadmium, Kupfer, Palladium, Nickel, Aluminium, Silicium, Zinn, Titan und Zirkon steht.

Bei einer solchen Ummetallierung lässt sich beispielsweise das 2,2-Difluorbenzodioxol-lithium mit Magnesiumbromid bzw. mit Zinkchlorid in das 2,2-Difluorbenzodioxol-magnesiumbromid bzw. in das 2,2-Difluorbenzodioxol-zinkchlorid überführen.

Es wurde nun überraschend gefunden, dass, ausgehend vom 2,2-Difluorbenzodioxol der Formel II das Gesamtverfahren zur Herstellung der Verbindungen der Formel I über die Stufe der Verbindungen der Formel III trotz der gänzlich unterschiedlichen Reaktanden, die nach und nach und unter möglicherweise wechselnden Bedingungen eingesetzt werden, gleichfalls ohne Isolierung weiterer intermediär entstehender Zwischenprodukte in einem Eintopfverfahren durchgeführt werden kann.

Gegenstand der vorliegenden Erfindung ist demgemäss auch ein Verfahren zur Herstellung von 3-(2,2-Difluorbenzodioxol-4-yl)-4-cyanopyrrol, wobei man in einer Eintopfreaktion bei -70°C bis +150°C 2,2-Difluorbenzodioxol in einem polaren Lösungsmittel oder in einem polaren Lösungsmittelgemisch mit einer Verbindung der Formel Me'ⁿY'ₙ gemäss Anspruch 2 in ein 4-Metallo-2,2-difluorbenzodioxol der Formel IIIa gemäss Anspruch 2 überführt, und dieses ohne Isolierung zunächst mit einem ungesättigten Nitril der Formel IV nach Anspruch 1, und dann mit einem Isocyanid der Formel V nach Anspruch 1 reagieren Läßt. Metallverbindungen der Formel III sind demnach für Verbindungen der Formel I die unmittelbaren Zwischenprodukte, die entweder als solche in das Herstellungsverfahren eingespeist werden oder in dessen erstem Verfahrensschritt entstehen.

Mit Hilfe des erfindungsgemässen Verfahrens kann das 3-(2,2-Difluorbenzodioxol-4-yl)-4-cyanopyrrol in hoher Ausbeute hergestellt werden.

Das für die einzelnen Reste R₁ und R₂ in der Formel IV als Bestandteil anderer Reste stehende C₁-C₄-Alkyl, kann verzweigt oder geradkettig sein: Es sind dies Methyl, Aethyl, Propyl, Isopropyl, Butyl, sek.Butyl, Isobutyl und tert.Butyl.

Der für R₁ stehende C₆-C₁₀-Aryloxy- oder C₆-C₁₀-Arylsulfonyloxyrest kann Phenoxy, C₁-C₄-Alkylphenoxy, α- oder β-Naphtyloxy, ferner Phenylsulfonyloxy, C₁-C₄-Alkylphenylsulfonyloxy oder α- oder β-Naphtylsulfonyloxy sein.

Als Beispiele für den unter R₃ in der Formel V genannten C₁-C₁₀ Kohlenwasserstoffrest können ohne Limitierung der Angaben Methyl, Ethyl, Isopropyl, Heptyl, Octyl, Cyclopentyl, Cyclohexyl, Decyl, Methylcyclohexyl, Phenyl, p-Toluyl, 1-Naphtyl oder 2-Naphthyl genannt werden.

Bei den Metallierungsreagenzien MeⁿYₙ für das 2,2-Difluorbenzodioxol steht, wie erwähnt, mindestens ein Y für ein stark basisches Anion. Dabei kann es sich um C₁-C₁₀Alkyl^{⊖}, bevorzugt C₁-C₄-Alkyl^{⊖}, um C₆-C₁₄Aryl^{⊖}, um C₁-C₁₀Alkoxy^{⊖}, bevorzugt C₁-C₄Alkoxy^{⊖}, um NH₂^{⊖}, C₁-C₁₀HydrocarbylNH^{⊖} oder um Di(C₁-C₁₀Hydrocarbyl)N^{⊖} oder auch um H^{⊖} handeln.

Bevorzugt werden unter diesen Anionen Alkyl^{⊖}, Aryl^{⊖} (wie Phenyl^{⊖}), Di(C₁-C₄Alkyl)N^{⊖}, (C₁-C₄Alkyl)(C₅-C₆Cycloalkyl)N^{⊖} oder Di(C₅-C₆Cycloalkyl)N^{⊖}. Für die Ummetallierung lassen sich auch Metallverbindungen mit Anionen wie Halogenid, Nitrat, Sulfat, Phosphat, Acetat, Formiat u.a. einsetzen.

Für die Herstellung der Verbindungen der Formel I in stufenweisen Verfahren oder im Eintopfverfahren eignen sich vor allem folgende Lösungsmittel:

Aliphatische oder aromatische Kohlenwasserstoffe, Ether, tertiäre Amine, N-alkylierte Säureamide, Lactame oder cyclische Harnstoffe, Sulfoxide, Sulfone, Nitrile, Ketone, Alkohole oder Mischungen hiervon. Beispiele sind Pentan, Isopentan, Hexan, Heptan, Cyclohexan, Methylcyclohexan, Benzol, Toluol, Xylol, Diethylether, Dibutylether, Tetrahydrofuran, Dioxan, Ethylenglykoldimethylether, Triethylamin, N,N,N',N'-Tetramethylethylendiamin, Hexamethylphosphorsäuretriamid, Dimethylformamid, Dimethylacetamid, N-Methylpyrrolidon, Ethylendimethylharnstoff, Propylendimethylharnstoff, Dimethylsulfoxid, Tetramethylensulfon, Acetonitril, Aceton, Methylethylketon, Methylisobutylketon, Cyclohexanon, Methanol, Ethanol, Isopropanol oder tert.Butanol.

Bei Verwendung unpolarer Lösungsmittel ist es zur Gewährleistung eines glatten Reaktionsverlaufs fast immer notwendig, ein Komplexierungsmittel zuzusetzen, bei Verwendung schwach polarer Lösungsmittel ist es zumindest vorteilhaft. Bezogen auf die Verbindung der Formel II wird das Komplexierungsmittel in einer Menge von 0,01 Mol-% bis zu 10-fachem Ueberschuss zugesetzt. Es eignen sich allgemein tertiäre Amine, N-substituierte Säureamide, Lactame, cyclische Harnstoffe, Alkoholate, Sulfoxide oder Ether, wie sie z.T. unter den vorgenannten Lösungsmitteln oder weiter oben unter den bevorzugten Komplexierungsmitteln bereits genannt wurden. Zu den Ethern sind im weiteren Sinne auch Kronenether zu zählen, z.B. 15-crown-5; 18-crown-6; Dibenzo-18-crown-6; Dicyclohexyl-18-crown-6; Dibenzo-24-crown-8; Dicyclohexyl-24-crown-8.

Die erfindungsgemässen Verfahren verlaufen bevorzugt unter Inertgas in Gegenwart einer komplexbildenden Verbindung (= Komplexierungsmittel) und in einem Lösungsmittel oder im Lösungsmittelgemisch.

Der bevorzugte erste Schritt bei jeder der vorstehend genannten Verfahren zur Herstellung der Verbindungen der Formel III, IIIa oder I ist die Metallierung des 2,2-Difluorbenzodioxols der Formel II mit Lithium oder bevorzugt einer lithiumorganischen Verbindung. Demgemäss ist von den Verbindungen der Formel III das 2,2-Difluorobenzodioxol-4-yl-lithium als Zwischenprodukt besonders bevorzugt.

Eine der bevorzugten Ausführungsformen des Gesamt-Verfahrens zur Herstellung von 3-(2,2-Difluorbenzodioxol-4-yl)-4-cyanopyrrol ist jene, bei welcher das in einem Kohlenwasserstoff gelöste 2,2-Difluor-1,3-benzodioxol bei -25° bis -5°C zu einem Gemisch aus ungefähr äquimolaren Mengen Tetramethylethylendiamin und n-Butyllithium gegeben wird, und dann das Reaktionsgemisch mit jeweils einer äquimolaren Menge von Ethoxymethylencyanessigsäureethylester und danach mit p-Toluolsulfonyl-methylisocyanid bei -25° bis +25°C versetzt wird. Als weiteres Lösungsmittel wird vorteilhaft Tetrahydrofuran verwendet. Als bevorzugtes Lösungsmittel für den letzten Reaktanden ist Methanol zu nennen.

Eine der insbesondere bevorzugten Ausführungsformen des Gesamtverfahrens zur Herstellung von 3-(2,2-Difluorbenzodiol-4-cyanopyrrol ist jene, bei welcher n-Butyllithium bei -25° bis -5°C zu einem in einem Kohlenwasserstoff gelösten Gemisch aus ungefähr equimolaren Mengen 2,2-Difluorbenzodioxol und Tetramethylethylendiamin gegeben wird, und dann das Reaktionsgemisch mit jeweils einer äquimolaren Menge von Ethoxymethylencyanessigsäureethylester und danach mit p-Toluolsulfonylmethylisocyanid bei -25° bis +25°C versetzt wird.

Die Organometall-Verbindungen der Formel III sind - sowohl in Lösung als auch in Suspension - in Form ihrer entsprechenden Komplexe bei niederen Temperaturen eine zeitlang stabil. Es ist bekannt, dass z.B. lithiumorganische Verbindungen oft als Dimere vorliegen. Zudem werden auch Lösungsmittel oder Komplexierungsmittel, die freie Elektronenpaare aufweisen, wie z.B. Ether oder tert.Amine, koordinativ an das Metall gebunden.

Bei der erfindungsgemässen Herstellung der Verbindungen der Formel III aus der Verbindung der Formel II sind als Reaktionspartner metallorganische Verbindungen hervorzuheben. Von den letzteren werden vor allem die lithiumorganischen Verbindungen, insbesondere Methyllithium, n-Butyllithium, sek.Butyllithium, tert.Butyllithium, Phenyllithium, Lithiumdiisopropylamid und Lithiumdicyclohexylamid bevorzugt.

Die zur Herstellung der Verbindungen der Formel III eingesetzten Metallverbindungen sowie die Verbindungen der Formeln II, IV und V sind bekannte und käufliche Produkte oder sind nach herkömmlichen Methoden herstellbar.

Das erfindungsgemässe Verfahren wird durch folgende Beispiele erörtert:

### Beispiel 1: Herstellung von 2,2-Difluor-1,3-benzodioxol-4-yl-lithium

a) Zu 69 ml (110 mMol) n-Butyllithium (1,6 molare Suspension in Toluol) werden unter Stickstoffatmosphäre bei -15° bis -10°C zunächst 13 g (110 mMol) N,N,N',N'-Tetramethylethylendiamin in 30 ml Toluol/Tetrahydrofuran (2:1), und dann 15,8 g (100 mMol) 2,2-Difluor-1,3-benzodioxol, in 120 ml Toluol/Tetrahydrofuran (2:1) gelöst, zugetropft, wobei n-Butan entsteht.
   Eine Probe des entstandenen gelösten Produkts der Formel III wird durch NMR-Spektroskopie identifiziert.
   - Signale δ ppm:: 6,83 d, (J = 7 Hz) entspricht: H 7
   7,10 dd (J = 7 Hz) entspricht: H 6
   7,80 d (J = 4 Hz) entspricht: H 5
b) Zu 29,5 g (254 mMol) N,N,N',N'-Tetramethylethylendiamin und 40,0 g (253 mMol) 2,2-Difluor-1,3-benzodioxol in 35 ml Toluol werden unter Stickstoffatmosphäre bei -15° bis -10°C 94,0 g (272 mMol) n-Butyllithium (18,5 % in Toluol) zugetropft, wobei neben der Titelverbindung n-Butan entsteht.
c) Zu 137,8 g (409 mMol) n-Butyllithiumlösung (19,0 % in Toluol) werden unter Stickstoffatmosphäre bei -15° bis -10°C 39,3 g (339 mMol) N,N,N',N'-Tetramethylethylendiamin zugetropft. Das entstandene Reaktionsgemisch wird bei -15° bis -10°C unter Stickstoffatmosphäre zu einer Lösung von 53,3 g (337 mMol) 2,2-Difluor-1,3-benzodioxol in 46 ml Toluol zugetropft, wobei neben der Titelverbindung n-Butan entsteht.

Folgende Verbindungen der Formel III werden analog Beispiel 1 hergestellt.

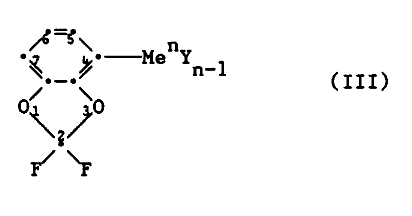

**Tabelle**

| Verbindungen der Formel III | | | | |
|---|---|---|---|---|
| Verbindung Nr. | Me | Y | NMR: δ (ppm) | Position H-Atom |
| 1 | Li | - | 6,83 (d) (J=7Hz) | 7 |
| | | | 7,10 (dd) (J=7Hz) | 6 |
| | | | 7,80 (d) (J=4Hz) | 5 |
| 2 | Na | - | | |
| 3 | K | - | 6,85 (d) (J=7Hz) | 7 |
| | | | 7,05 (dd) (J=7Hz) | 6 |
| | | | 7,76 (d) (J=4Hz) | 5 |
| 4 | Cs | Cl⁻ | | |
| 5 | Mg | Br⁻ | 6,86 (d) (J=7Hz) | 7 |
| | | | 6,65 (dd) (J=7Hz) | 6 |
| | | | 7,42 (d) (J=4Hz) | 5 |
| 6 | Hg | Cl⁻ | 6,93 (d) (J=7Hz) | 7 |
| | | | 6,84 (dd) (J=7Hz) | 6 |
| | | | 7,05 (d) (J=4Hz) | 5 |
| 7 | Zn | Cl⁻ | 6,84 (d) (J=7Hz) | 7 |
| | | | 6,98 (dd) (J=7Hz) | 6 |
| | | | 7,14 (d) (J=4Hz) | 5 |
| 8 | Cu | Cl⁻ | | |

### Beispiel 2: Herstellung von 3-(2,2-Difluorbenzodioxol-4-yl)-4-cyanopyrrol

a) Zu 261 ml 1,6 N n-Butyllithium in Hexan werden unter Inertgasatmosphäre bei -20° zunächst 48,2 g N,N,N',N'-Tetramethylethylendiamin, dann nach 30 Min. Nachrühren 60 g 2,2-Difluor-1,3-benzodioxol, gelöst in 200 ml Hexan gegeben. Nach 15 Min. werden 70,7 g Ethoxymethylencyanessigsäureethylester, gelöst in 250 ml Tetrahydrofuran, bei -15° innerhalb von 25 Min. zugegeben. Die resultierende Suspension wird nach weiteren 30 Minuten bei 0° während 25 Minuten mit 81,5 g p-Toluolsulfonylmethylisocyanid, gelöst in 220 ml Tetrahydrofuran versetzt. Die Lösung wird anschliessend auf 25° erwärmt, und es werden 400 ml Lösungsmittel im Vakuum verdampft.
   Der Rückstand wird mit 500 ml Essigsäureethylester verdünnt, die entstandene Lösung wird je zweimal mit 300 ml Wasser und 300 ml gesättigter Kochsalzlösung gewaschen und über Magnesiumsulfat getrocknet. Das Lösungsmittel wird im Vakuum verdampft.
   Der Rückstand wird über Kieselgel mit Hexan/Essigsäureethylester (3:1) als Eluiermittel chromatographiert.
   Nach Verdampfen des Lösungsmittels im Vakuum erhält man die Titelverbindung mit Schmelzpunkt = 193-195°C. Ausbeute 70 % der Theorie, bezogen auf 2,2-Difluorbenzo-1,3-dioxol.
b) Zu 29,5 g (254 mMol) N,N,N',N'-Tetramethylethylendiamin und 40 g (253 mMol) 2,2-Difluor-1,3-benzodioxol in 35 ml Toluol werden unter Stickstoffatmosphäre bei -15° bis -10°C 106,2 g (307 mMol) n-Butyllithiumlösung (18,5 % in Toluol) zugetropft. Anschliessend werden 52,1 g (308 mMol) Ethoxymethylencyanessigsäureethylester in 115 ml Toluol bei -15° bis -10°C innert 2 Stunden zugegeben. Nach 20 Min. wird die entstandene Suspension auf 0°C erwärmt und während 30 Min. werden 50 g (256 mMol) p-Toluolsulfonylmethylisocyanid, gelöst in 135 ml Tetrahydrofuran, addiert. Das Reaktionsgemisch wird anschliessend auf 25°C erwärmt, auf 150 ml Wasser gegeben und filtriert, wobei die Titelverbindung erhalten wird.
c) Zu 137,8 g (409 mMol) n-Butyllithiumlösung (19,0 % in Toluol) werden unter Stickstoffatmosphäre bei -15° bis -10°C 39,3 g (339 mMol) N,N,N',N'-Tetramethylethylendiamin zugetropft. Das entstandene Reaktionsgemisch wird bei -15° bis -10°C unter Stickstoffatmosphäre zu einer Lösung von 53,3 g (337 mMol) 2,2-Difluorbenzo-2,3-dioxol in 46 ml Toluol zudosiert. Anschliessend werden 69,1 g (409 mMol) Ethoxymethylencyanessigsäureethylester in 150 ml Toluol bei -15° bis -10°C zudosiert. Die entstandene Suspension wird danach auf 0°C erwärmt und es werden 66,6 g (342 mMol) p-Toluolsulfonylmethylisocyanid, gelöst in 180 ml Tetrahydrofuran, addiert. Das Reaktionsgemisch wird anschliessend auf 25°C erwärmt, auf 200 ml Wasser gegeben und filtriert, wobei die Titelverbindung erhalten wird.

## Patentansprüche

1. Verfahren zur Herstellung von 3-(2,2-Difluorbenzodioxol-4-yl)-4-cyanopyrrol der Formel I 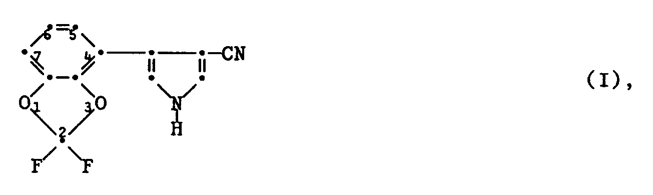 dadurch gekennzeichnet, dass man ein 4-Metallo-2,2-difluorbenzodioxol der Formel III, wobei Me ein Metall ausgewählt aus der Gruppe bestehend aus Li, Na, K, Cs, Mg, Ca, Zn, Cd, Cu, Pd, Ni, Al, Si, Sn, Ti und Zr, n die Wertigkeit dieses Metalls und Y einen anionischen Rest, ausgewählt aus der Gruppe bestehend aus Halogenid, Nitrat, Sulfat, Phosphat, Acetat, Formiat, C₁-C₁₀-Alkyl^{⊖}, C₆-C₁₄-Aryl^{⊖}, C₁-C₁₀-Alkoxy^{⊖}, NH₂^{⊖}, C₁-C₁₀-HydrocarbylNH^{⊖}, Di(C₁-C₁₀-hydrocarbyl)N^{⊖} und H^{⊖} bedeuten, in einem polaren Lösungsmittel oder in einem polaren Lösungsmittelgemisch, zunächst mit einem ungesättigten Nitril der Formel IV zur Reaktion bringt, 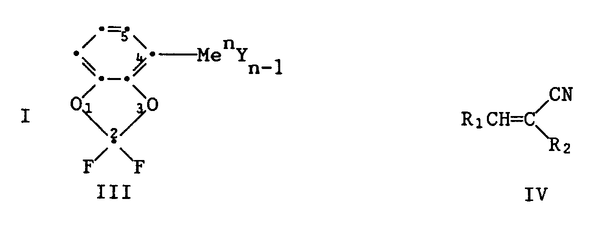 worin
R₁ Halogen, C₁-C₄-Alkoxy, C₆-C₁₀-Aryloxy, Di-C₁-C₄-alkylamino, C₁-C₄-Alkylsulfonyloxy, C₆-C₁₀-Arylsulfonyloxy oder C₁-C₄-Alkylcarbonyloxy und
R₂ einen C₁-C₄-Alkoxycarbonylrest bedeuten, und dann mit einem Isocyanid der Formel V reagieren lässt,
R₃-SO₂CH₂NC, (V),
worin R₃ ein offenkettiger oder cyclischer, aliphatischer oder aromatischer C₁-C₁₀-Kohlenwasserstoffrest ist.

2. Verfahren zur Herstellung von Verbindungen der Formel IIIa dadurch gekennzeichnet, dass man 2,2-Difluorbenzodioxol der Formel II, in einem polaren Lösungsmittel oder in einem polaren Lösungsmittelgemisch mit einer Metallverbindung Me'ⁿY'ₙ bei -70°C bis +150°C zur Reaktion bringt, 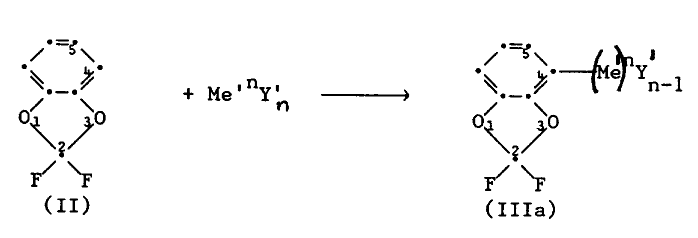 wobei Me' ein Metall ausgewählt aus der Gruppe bestehend aus Li, Na, K, Cs, Mg und Ca, n die Wertigkeit dieses Metalls und Y' einen anionischen Rest ausgewählt aus der Gruppe bestehend aus C₁-C₁₀-Alkyl^{⊖}, C₆-C₁₄-Aryl^{⊖}, NH₂^{⊖}, C₁-C₁₀-HydrocarbylNH^{⊖}, Di(C₁-C₁₀-hydrocarbyl)N^{⊖} und H^{⊖}, bedeuten.

3. Verfahren zur Herstellung von 3-(2,2-Difluorbenzodioxol-4yl)-4-cyanopyrrol, dadurch gekennzeichnet, dass man in einer Eintopfreaktion bei -70°C bis +150°C 2,2-Difluorbenzodioxol in einem polaren Lösungsmittel oder in einem polaren Lösungsmittelgemisch mit einer Verbindung der Formel Me'ⁿY'ₙ gemäss Anspruch 2 in ein 4-Metallo-2,2-difluorbenzodioxol der Formel IIIa gemäss Anspruch 2 überführt, und dieses ohne Isolierung zunächst mit einem ungesättigten Nitril der Formel IV nach Anspruch 1, und dann mit einem Isocyanid der Formel V nach Anspruch 1 reagieren lässt.

4. Verfahren zur Herstellung von Verbindungen der Formel III nach Anspruch 1, dadurch gekennzeichnet, dass man eine Verbindung der Formel IIIa gemäss Anspruch 2 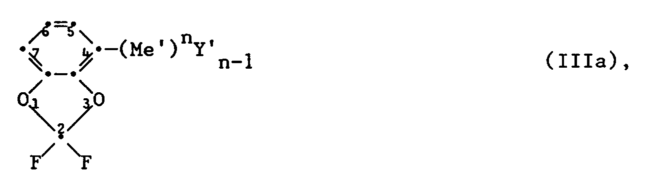 mit einer Verbindung der Formel MeⁿYₙ umsetzt, worin Me, Y und n die in Anspruch 1 gegebenen Bedeutungen haben, mit der Massgabe, dass Me von Me' verschieden ist.

5. Verfahren nach Anspruch 3, dadurch gekennzeichnet, dass man die Reaktion unter Inertgas-Atmosphäre in einem Lösungsmittel, ausgewählt aus einer Gruppe bestehend aus aliphatischen und aromatischen Kohlenwasserstoffen, Aethern, Ketonen und dipolaren Lösungsmitteln durchführt.

6. Verfahren nach Anspruch 5, dadurch gekennzeichnet, dass man die Metallierung und die folgenden Umsetzungen zwischen -70° und +150°C durchführt.

7. Verfahren nach Anspruch 6, dadurch gekennzeichnet, dass man zwischen -25° und +80°C arbeitet.

8. Verfahren nach Anspruch 3, dadurch gekennzeichnet, dass ein polares Lösungsmittelgemisch verwendet wird, das aus mindestens zwei Komponenten besteht, und worin mindestens eine dieser Komponenten eine Verbindung aus einer Gruppe bestehend aus Ethern, tert.Aminen, cyclischen Harnstoffen und N-substituierten Säureamiden ausgewählt wird.

9. Verfahren nach Anspruch 8, dadurch gekennzeichnet, dass mindestens eine Komponente des Lösungsmittelgemisches eine Verbindung aus einer Gruppe bestehend aus Ethylenglykoldimethylether, Hexamethylphosphorsäuretriamid, 1,3-Dimethyl-2-imidazolon, Dimethylethylenharnstoff, Dimethylpropylenharnstoff und N,N,N',N'-Tetramethylethylendiamin ausgewählt wird und dass als Metallverbindung eine lithiumorganische Verbindung eingesetzt wird.

10. Verfahren nach Anspruch 9, dadurch gekennzeichnet, dass man das in einem Kohlenwasserstoff gelöste 2,2-Difluor-1,3-benzodioxol zu einem Gemisch aus äquimolaren Mengen Tetramethylethylendiamin und Butyllithium bei -25 bis -5°C gibt und das Reaktionsgemisch nacheinander jeweils mit äquimolaren Mengen von Ethoxymethylencyanessigsäureaethylester und danach mit p-Toluolsulfonyl-methylisocyanid zwischen -25 und +25°C umsetzt.

11. Verfahren nach Anspruch 9, dadurch gekennzeichnet, dass man n-Butyllithium bei -25° bis -5° zu einem in einem Kohlenwasserstoff gelösten Gemisch aus equimolaren Mengen 2,2-Difluor-1,3-benzodioxol und Tetramethylethylendiamin gibt und das Reaktionsgemisch nacheinander jeweils mit equimolaren Mengen von Ethoxymethylencyanessigsäureethylester und danach mit p-Toluol-sulfonyl-methylisocyanid zwischen -25° und +25°C umsetzt.

12. Die nach einem der Verfahren gemäss Anspruch 2 oder Anspruch 4 herstellbaren Verbindungen der Formel III oder IIIa als Lösung oder als Suspension in einem polaren Lösungsmittel oder in einem polaren Lösungsmittelgemisch, mit der Massgabe, dass Me bzw. Me' nicht Silicium ist.

13. 2,2-Difluor-1,3-benzodioxol-4-yl-lithium gemäss Anspruch 12.

14. 2,2-Difluor-1,3-benzodioxol-4-yl-lithium gemäss Anspruch 13, worin das Lösungsmittelgemisch aus Toluol, Tetrahydrofuran und N,N,N',N'-Tetramethylethylendiamin oder aus Toluol und N,N,N',N'-Tetramethylethylendiamin besteht.

15. Verfahren nach Anspruch 2, dadurch gekennzeichnet, dass man als Metallverbindung MeⁿYₙ eine lithiumorganische Verbindung einsetzt.

16. Verfahren nach Anspruch 15, dadurch gekennzeichnet, dass man eine lithiumorganische Verbindung, ausgewählt aus einer Gruppe bestehend aus Methyllithium, n-Butyllithium, sek.Butyllithium, tert.Butyllithium, Phenyllithium, Lithiumdiisopropylamid und Lithiumdicyclohexylamid einsetzt.

17. Verfahren nach Anspruch 16, dadurch gekennzeichnet, dass die lithiumorganische Verbindung n-Butyllithium ist.

## Claims

1. A process for the preparation of 3-(2,2-difluorobenzodioxol-4-yl)-4-cyanopyrrole of formula I 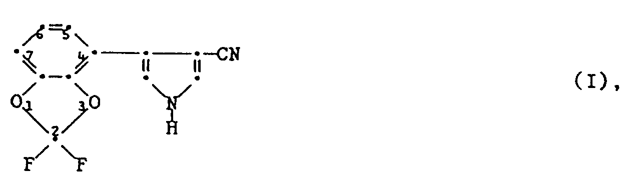 wherein a 4-metallo-2,2-difluorobenzodioxole of formula III, wherein Me is a metal selected from the group consisting of Li, Na, K, Cs, Mg, Ca, Zn, Cd, Cu, Pd, Ni, Al, Si, Sn, Ti and Zr, n is the valency of that metal and Y is an anionic radical selected from the group consisting of halide, nitrate, sulfate, phosphate, acetate, formate, C₁-C₁₀alkyly^{⊖}, C₆-C₁₄aryl^{⊖}, C₁-C₁₀alkoxy^{⊖}, NH₂^{⊖}, C₁-C₁₀hydrocarbylNH^{⊖}, di(C₁-C₁₀hydrocarbyl)N^{⊖} and H^{⊖}, is reacted in a polar solvent or in a polar solvent mixture first with an unsaturated nitrile of formula IV 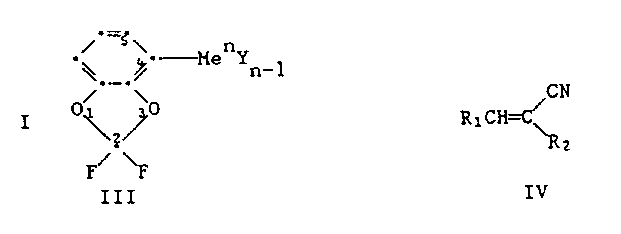 wherein
R₁ is halogen, C₁-C₄alkoxy, C₆-C₁₀aryloxy, di-C₁-C₄alkylamino, C₁-C₄alkylsulfonyloxy, C₆-C₁₀arylsulfonyloxy or C₁-C₄alkylcarbonyloxy and
R₂ is a C₁-C₄alkoxycarbonyl radical,
and then with an isocyanide of formula V
R₃-SO₂CH₂NC (V),
wherein
R₃ is an open-chain or cyclic, aliphatic or aromatic C₁-C₁₀hydrocarbon radical.

2. A process for the preparation of a compound of formula IIIa, wherein 2,2-difluorobenzodioxole of formula II is reacted in a polar solvent or in a polar solvent mixture with a metal compound Me'ⁿY'ₙ at from -70°C to +150°C 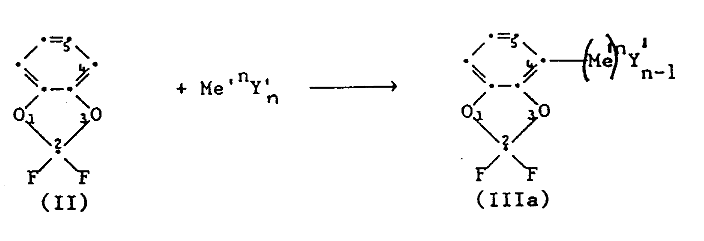 wherein Me' is a metal selected from the group consisting of Li, Na, K, Cs, Mg and Ca, n is the valency of that metal and Y' is an anionic radical selected from the group consisting of C₁-C₁₀alkyl^{⊖}, C₆-C₁₄aryl^{⊖}, NH₂^{⊖}, C₁-C₁₀hydrocarbylNH^{⊖}, di(C₁-C₁₀hydrocarbyl)N^{⊖} and H^{⊖}.

3. A process for the preparation of 3-(2,2-difluorobenzodioxol-4-yl)-4-cyanopyrrole, wherein, in a one-pot reaction, 2,2-difluorobenzodioxole is converted at from -70°C to +150°C in a polar solvent or in a polar solvent mixture with a compound of formula Me'ⁿY'ₙ according to claim 2 into a 4-metallo-2,2-difluorobenzodioxole of formula IIIa according to claim 2 which is reacted, without being isolated, first with an unsaturated nitrile of formula IV according to claim 1 and then with an isocyanide of formula V according to claim 1.

4. A process for the preparation of a compound of formula III according to claim 1, wherein a compound of formula IIIa according to claim 2 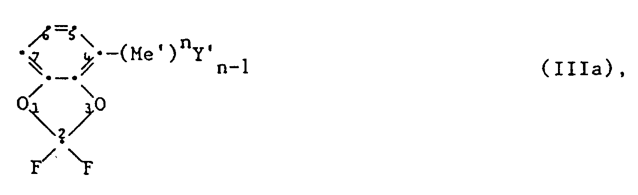 is reacted with a compound of formula MeⁿYₙ wherein Me, Y and n are as defined in claim 1, with the proviso that Me is different from Me'.

5. A process according to claim 3, wherein the reaction is carried out under an inert gas atmosphere in a solvent selected from a group consisting of aliphatic and aromatic hydrocarbons, ethers, ketones and dipolar solvents.

6. A process according to claim 5, wherein the metallation and the subsequent reactions are carried out at from -70° to +150°C.

7. A process according to claim 6, wherein the operation is carried out at from -25° to +80°C.

8. A process according to claim 3, wherein a polar solvent mixture is used that consists of at least two components, and wherein at least one of those components is a compound from a group consisting of ethers, tert-amines, cyclic ureas and N-substituted acid amides.

9. A process according to claim 8, wherein at least one component of the solvent mixture is a compound from a group consisting of ethylene glycol dimethyl ether, hexamethylphosphoric acid triamide, 1,3-dimethyl-2-imidazolone, dimethylethyleneurea, dimethylpropyleneurea and N,N,N',N'-tetramethylethylenediamine, and an organolithium compound is used as the metal compound.

10. A process according to claim 9, wherein 2,2-difluoro1,3-benzodioxole dissolved in a hydrocarbon is added to a mixture of equimolar amounts of tetramethylethylenediamine and butyllithium at from -25 to -5°C and the reaction mixture is reacted in succession with an equimolar amount of ethoxymethylenecyanoacetic acid ethyl ester and then with an equimolar amount of p-toluenesulfonylmethyl isocyanide at from -25 to +25°C.

11. A process according to claim 9, wherein n-butyllithium is added at from -25° to -5° to a mixture, dissolved in a hydrocarbon, of equimolar amounts of 2,2-difluoro-1,3-benzodioxole and tetramethylethylenediamine and the reaction mixture is reacted in succession with an equimolar amount of ethoxymethylenecyanoacetic acid ethyl ester and then with an equimolar amount of p-toluenesulfonylmethyl isocyanide at from -25° to +25°C.

12. A compound of formula III or IIIa, which can be prepared by a process according to claim 2 or claim 4, in the form of a solution or a suspension in a polar solvent or in a polar solvent mixture, with the proviso that Me and Me' are not silicon.

13. 2,2-difluoro-1,3-benzodioxol-4-yl-lithium according to claim 12.

14. 2,2-difluoro-1,3-benzodioxol-4-yl-lithium according to claim 13, wherein the solvent mixture comprises toluene, tetrahydrofuran and N,N,N'N'-tetramethylethylenediamine, or toluene and N,N,N',N'-tetramethylethylenediamine.

15. A process according to claim 2, wherein an organolithium compound is used as the metal compound MeⁿYₙ.

16. A process according to claim 15, wherein an organolithium compound selected from a group consisting of methyllithium, n-butyllithium, sec-butyllithium, tert-butyllithium, phenyllithium, lithium diisopropylamide and lithium dicyclohexylamide is used.

17. A process according to claim 16, wherein the organolithium compound is n-butyllithium.

## Revendications

1. Procédé pour la préparation du 3-(2,2-difluorobenzodioxol-4-yl)-4-cyanopyrrole de formule I 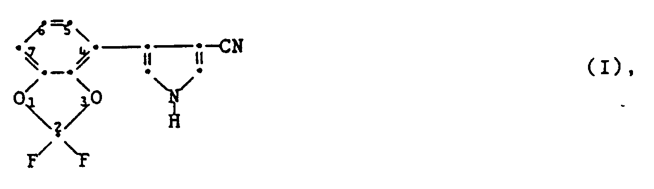 caractérisé en ce que l'on fait réagir un 4-métallo-2,2-difluorobenzodioxole de formule III où Me représente un métal choisi dans le groupe constitué par Li, Na, K, Cs, Mg, Ca, Zn, Cd, Cu, Pd, Ni, AI, Si, Sn, Ti et Zr, n représente la valence de ce métal et Y un reste anionique choisi dans le groupe constitué par un halogénure, le nitrate, le sulfate, le phosphate, l'acétate, le formiate, un alkyle^{⊖} en C₁-C₁₀, un aryle^{⊖} en C₆-C₁₄, un alcoxy^{⊖} en C₁-C₁₀, NH₂^{⊖} , un hydrocarbyle en C₁-C₁₀ NH^{⊖}, un di(hydroxycarbyle en C₁-C₁₀) N^{⊖} et H^{⊖}, dans un solvant polaire ou dans un mélange de solvants polaires, tout d'abord avec un nitrile insaturé de formule IV 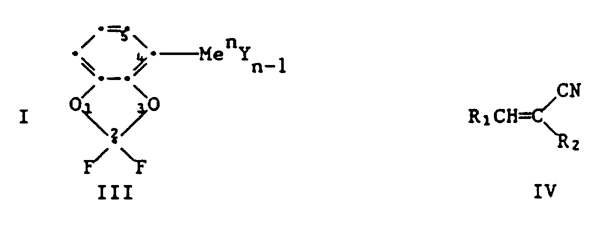 où
R₁ représente un halogène, un alcoxy en C₁-C₄, un aryloxy en C₆-C₁₀, un dialkyle en C₁-C₄ amino, un alkyle en C₁-C₄ sulfonyloxy, un aryle en C₆-C₁₀ sulfonyloxy ou un alkyle en C₁-C₄ carbonyloxy
et
R₂ représente un reste alcoxy en C₁-C₄ carbonyle, puis avec un isocyanure de formule V
R₃-SO₂CH₂NC (V)
où R₃ est un reste hydrocarboné en C₁-C₁₀ aliphatique ou aromatique, à chaîne ouverte ou cyclique.

2. Procédé pour la préparation des composés de formule IIIa, caractérisé en ce que l'on fait réagir le 2,2-difluorobenzodioxole de formule II dans un solvant polaire ou dans un mélange de solvants polaires, avec un composé métallique Me'ⁿY'ₙ , à une température comprise entre -70°C et +150°C 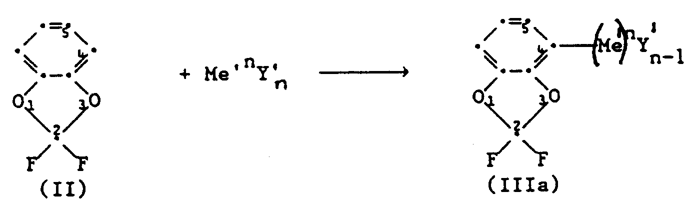 où Me' représente un métal choisi dans le groupe constitué par Li, Na, K, Cs, Mg et Ca, n représente la valence de ce métal et Y' un reste anionique choisi dans le groupe constitué par un alkyle^{⊖} en C₁-C₁₀, un aryle^{⊖} en C₆-C₁₄, NH₂^{⊖} , un hydrocarbyle en C₁-C₁₀ NH^{⊖} , un di(hydroxycarbyle en C₁-C₁₀) N^{⊖} et H^{⊖}.

3. Procédé pour la préparation du 3-(2,2-difluorobenzodioxol-4-yl)-4-cyanopyrrole, caractérisé en ce que l'on transforme dans une réaction "en un pot unique" à une température comprise entre -70°C et +150°C, le 2,2-difluorobenzodioxole dans un solvant polaire ou dans un mélange de solvants polaires avec un composé de formule Me'ⁿY'ₙ selon la revendication 2 en un 4-métallo-2,2-difluorobenzodioxole de formule IIIa selon la revendication 2 et en ce que l'on fait réagir celui-ci, sans l'avoir isolé, tout d'abord avec un nitrile insaturé de formule IV selon la revendication 1, puis avec un isocyanure de formule V selon la revendication 1.

4. Procédé pour la préparation des composés de formule III selon la revendication 1, caractérisé en ce que l'on fait réagir un composé de formule IIIa selon la revendication 2 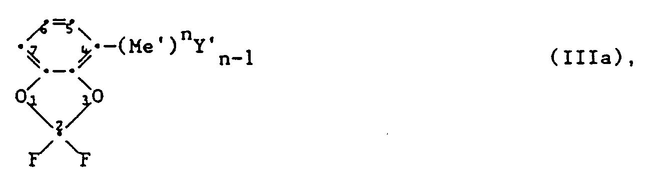 avec un composé de formule MeⁿYₙ où Me, Y et n ont les significations données dans la revendication 1, sous réserve que Me soit différent de Me'.

5. Procédé selon la revendication 3, caractérisé en ce que l'on effectue la réaction, sous une atmosphère de gaz inerte, dans un solvant choisi dans le groupe constitué par des hydrocarbures aliphatiques et aromatiques, des éthers, des cétones et des solvants dipolaires.

6. Procédé selon la revendication 5, caractérisé en ce que l'on effectue la métallation et les réactions suivantes à une température comprise entre -70° et +150°C.

7. Procédé selon la revendication 6, caractérisé en ce que l'on opère à une température comprise entre -25° et +80°C.

8. Procédé selon la revendication 3, caractérisé en ce que l'on utilise un mélange de solvants polaires constitué d'au moins deux composants et dans lequel au moins l'un de ces composants est un composé choisi dans le groupe constitué par des éthers, des amines tertiaires, des urées cycliques et des amides N-substitués.

9. Procédé selon la revendication 8, caractérisé en ce qu'au moins l'un des composants du mélange de solvants est un composé choisi dans le groupe constitué par l'éthylèneglycoldiméthyléther, l'hexaméthylphosphorotriamide, la 1,3-diméthyl-2-imidazolone, la diméthyléthylèneurée, la diméthylpropylèneurée et la N,N,N',N'-tétraméthyléthylènediamine et en ce que l'on utilise comme composé métallique un composé organolithique.

10. Procédé selon la revendication 9, caractérisé en ce que l'on ajoute le 2,2-difluoro-1,3-benzodioxole dissous dans un hydrocarbure à un mélange constitué en quantités équimolaires de tétraméthyléthylènediamine et de butyllithium, à une température comprise entre -25 et -5°C, et en ce que l'on fait réagir le mélange réactionnel successivement avec des quantités équimolaires de l'ester éthylique de l'acide éthoxyméthylènecyanacétique, puis avec l'isocyanure de p-toluènesulfonylméthyle à une température comprise entre -25 et +25°C.

11. Procédé selon la revendication 9, caractérisé en ce que l'on ajoute le n-butyllithium, à une température comprise entre -25 et -5°C, à un mélange dissous dans un hydro carbure, constitué en quantités équimolaires de 2,2-difluoro-1,3-benzodioxole et de tétraméthyléthylènediamine et en ce que l'on fait réagir le mélange réactionnel successivement avec des quantités équimolaires de l'ester éthylique de l'acide éthoxyméthylènecyanacétique, puis avec l'isocyanure de p-toluènesulfonylméthyle, à une température comprise entre -25 et +25°C.

12. Les composés de formule III ou IIIa pouvant être obtenus par l'un des procédés selon la revendication 2 ou 4, comme solution ou comme suspension, dans un solvant polaire ou dans un mélange de solvants polaires, sous réserve que Me ou Me' ne soit pas le silicium.

13. Le 2,2-difluoro-1,3-benzodioxol-4-yl-lithium selon la revendication 12.

14. Le 2,2-difluoro-1,3-benzodioxol-4-yl-lithium selon la revendication 13 où le mélange de solvants est constitué de toluène, de tétrahydrofuranne et de N,N,N',N'-tétraméthyléthylènediamine ou de toluène et de N,N,N',N'-tétraméthyléthylènediamine.

15. Procédé selon la revendication 2, caractérisé en ce que l'on utilise comme composé métallique MeⁿYₙ un composé organolithique.

16. Procédé selon la revendication 15, caractérisé en ce que l'on utilise un composé organolithique choisi dans le groupe constitué par le méthyllithium, le n-butyllithium, le sec-butyllithium, le tert-butyllithium, le phényllithium, le lithiumdiisopropylamide et le lithiumdicyclohexylamide.

17. Procédé selon la revendication 16, caractérisé en ce que le composé organolithique est le n-butyllithium.
